# EUROPEAN PATENT APPLICATION

(11) **EP 3 758 014 A1**
(43) Date of publication of application: **30.12.2020**
(21) Application number: 19182266.7
(22) Date of filing: 25.06.2019
(51) Int. Cl.: G16H 20/60

(54) **FOOD PROCESSING FOR DYSPHAGIA PATIENTS**

(71) Applicant: Koninklijke Philips N.V., 5656 AG Eindhoven (NL)
(72) Inventor: TANG, Jiani (Jennie), 5656 AE Eindhoven (NL)
(74) Representative: de Haan, Poul Erik

(57) **Abstract**

A food processing apparatus (10) for preparing a target fluid food product is disclosed that is arranged to receive (103) a list (1) of amounts of ingredients for preparing the target fluid food product from a user interface, determine a fluid thickness score for the target fluid food product based on a comparison of the received list of amounts of ingredients with at least some of the fluid food product recipes in said database; compare the determined fluid thickness score for the target fluid food product against a critical fluid thickness score, and, if said comparison is indicative of the fluid thickness of the target fluid food product being below a critical thickness for said target fluid food product, determine at least one alteration to the received list of amounts of ingredients for increasing the fluid thickness score of the target fluid food product to at least the critical fluid thickness score; and communicate the at least one determined alteration to the received list of amounts of ingredients to the user interface. A method of controlling such a food processing apparatus is also disclosed.

## Description

### FIELD OF THE INVENTION

The present invention relates to a food processing apparatus for preparing a target fluid food product comprising a controller for controlling the food processing apparatus, said controller in operation being responsive to a user interface for said food processing apparatus and having access to a database storing a plurality of fluid food product recipes.

The present invention further relates to a method of controlling such a food processing apparatus.

### BACKGROUND OF THE INVENTION

Dysphagia is a debilitating condition in which the sufferer from this condition has difficulty swallowing food products including liquids. This can cause coughing or choking when eating or drinking, bringing food back up, a sensation that food is stuck in the throat, amongst other symptoms. It is estimated that about 8% of the world's population (590 million people) suffers from dysphagia, which can lead to malnutrition and pneumonia as the most negative health impacts.

In order to manage this condition, it is important to manage the thickness of the food products consumed by dysphagia sufferers, such as the thickness of fluids consumed by such sufferers. In this context, the International Dysphagia Diet Standardisation Initiative (IDDSI) has developed a framework in which drinks and foods are categorized by thickness scores, with drinks having scores ranging from 0-4 and foods having scores ranging from 3-7. A higher score is indicative of a higher thickness or chewiness of the food product. For example, for drinks, a score of 0 indicates a thin liquid, a score of 1 indicates a slightly thick liquid, a score of 2 indicates a mildly thick liquid, a score of 3 indicates a moderately thick liquid and a score of 4 indicates an extremely thick liquid.

For dysphagia patients, it is important to know which thickness score corresponds to a food product that can be tolerated by the patient without suffering from (severe) dysphagia symptoms. US 2017/027495 A1 discloses a technique for determining a swallowing deficiency using a device for assessing the characteristics of the swallowing process in a subject a sensor that is capable of detecting vibrations of the throat during swallowing. The device forms part of a kit including a thickening agent to be added to a fluid such as water in order to produce fluids having a particular thickness score. By having a patient drinking fluids with different particular thickness scores, the optimal fluid thickness score at which the patient can comfortably consume such a fluid can be determined with the device. However, it is difficult for such patients to prepare a particular liquidized or fluid food product that has an appropriate fluid thickness score for them, e.g. a thickness that is sufficiently high such as to minimize dysphagia symptoms when consuming the fluid food product.

### SUMMARY OF THE INVENTION

The present invention seeks to provide a food processing apparatus that can provide a dysphagia sufferer with a processed food product having an appropriate fluid thickness for said sufferer.

The present invention further seeks to provide a method of controlling such a food processing apparatus.

According to an aspect, there is provided a food processing apparatus for preparing a target fluid food product comprising a controller for controlling the food processing apparatus, said controller in operation being responsive to a user interface for said food processing apparatus and having access to database storing a plurality of fluid food product recipes, each of said recipes being associated with a fluid thickness score for the fluid food product to be made from said recipe, the controller implementing a set of algorithms configured to receive a list of amounts of ingredients for preparing the target fluid food product from the user interface; determine a fluid thickness score for the target fluid food product based on a comparison of the received list of amounts of ingredients with at least some of the fluid food product recipes in said database; compare the determined fluid thickness score for the target fluid food product against a critical fluid thickness score, and, if said comparison is indicative of the fluid thickness of the target fluid food product being below a critical thickness for said target fluid food product: determine at least one alteration to the received list of amounts of ingredients for increasing the fluid thickness score of the target fluid food product to at least the critical fluid thickness score; and communicate the at least one determined alteration to the received list of amounts of ingredients to the user interface.

With such a food processing apparatus, a user may specify a recipe of a fluid food product such as a smoothie, soup or the like to be prepared by the food processing apparatus without having to worry about whether the liquid food product has the desired fluid thickness score in accordance with the level of dysphagia the user suffers from, because the food processing apparatus, upon determining that the expected fluid thickness score for the target fluid food product deviates from the target fluid thickness score for the user, will automatically generate suggested one or more possible amendments to the recipe of the fluid food product to rectify this deviation, thereby allowing the user to adjust the expected fluid thickness score of the fluid food product by amending its recipe such that the thickness of the fluid food product to be prepared is more in line with the optimal thickness at which the dysphagia symptoms of the user are minimized.

In an embodiment, the controller is further configured to train the set of algorithms to determine a fluid thickness score for the target fluid food product from the received list of amounts of ingredients based on the plurality of fluid thickness scores for the fluid food product recipes in said database. This for example may be achieved with common machine learning techniques, and has the advantage that the accuracy of the determination of the fluid thickness score for the target fluid food products may be improved over time.

In a further embodiment, the controller is further configured to receive user feedback information from said user interface on at least one of the fluid thickness score and a taste score of the target fluid food product produced based on the list of amounts of ingredients including at least one of said alterations to said list; and train the set of algorithms based on the received user feedback. This again for example may be achieved with common machine learning techniques, and also has the advantage that the accuracy of the determination of the fluid thickness score for the target fluid food products and/or the taste of the food products prepared using altered recipes may be improved over time.

Such proposed alterations to the recipe of a target food product may include a change in a ratio of solid and liquid ingredients in said list in order to adjust the expected fluid thickness of the target food product. Alternatively or additionally, such proposed alterations to the recipe of a target food product may include at least one of a replacement of at least part of an amount of a first liquid ingredient in said received list with a further amount of a further liquid ingredient having a higher thickness score than said first liquid ingredient; and a replacement of at least part of an amount of a first solid ingredient in said received list with a further amount of a further solid ingredient having a higher fat content and/or a higher starch content than said first solid ingredient or an addition of said further amount of the further solid ingredient to said received list of amounts of ingredients.

The controller may be further adapted to directly or indirectly obtain said critical fluid thickness score from the user interface. This has the advantage that a user can specify his or her optimal fluid thickness score in a convenient manner to the food processing apparatus.

To this end, the food processing apparatus may further comprise a further database of user identifiers each associated with a critical fluid thickness score for said user, wherein the controller is adapted to retrieve the critical fluid thickness from said further database based on a user identifier received from the user interface. This has the further advantage that different users may utilize the same food processing apparatus, in which each user may be identified by simply providing his or her user identifier.

The food processing apparatus may further comprise said user interface and/or said database. Alternatively or additionally, the food processing apparatus may further comprise a communication interface communicatively coupled to the controller for communicating with at least one of the user interface and the database. In other words, the user interface and/or the database of recipes do not have to form an integral part of the food processing apparatus. For example, in the case of the user interface, this has the advantage that the food processing apparatus maybe controlled remotely, e.g. through a mobile communications device implementing the user interface, which allows for advantageous scenarios such as a user shopping for ingredients and verifying the list of ingredients with the food processing apparatus to ensure that the target food product to be prepared with the food processing apparatus has the desired fluid thickness, such that the user can purchase the correct ingredients and the correct amounts of such ingredients based on the feedback provided by the food processing apparatus.

According to another aspect, there is provided a method of controlling a food processing apparatus for preparing a target fluid food product, the method comprising receiving a list of amounts of ingredients for preparing the target fluid food product from a user interface for said food processing apparatus; determining a fluid thickness score for the target fluid food product based on a comparison of the received list of amounts of ingredients with at least some fluid food product recipes in a database storing a plurality of said fluid food product recipes, each of said recipes being associated with a fluid thickness score for the fluid food product to be made from said recipe; comparing the determined fluid thickness score for the target fluid food product against a critical fluid thickness score, and, if said comparison is indicative of the fluid thickness of the target fluid food product being below a critical thickness for said target fluid food product: determining at least one alteration to the received list of amounts of ingredients for increasing the fluid thickness score of the target fluid food product to at least the critical fluid thickness score; and communicating the at least one alteration to the received list of amounts of ingredients to the user interface. With such a method, it can be ensured that a food product prepared in accordance with a given recipe has the appropriate fluid thickness score for its consumer, thereby minimizing dysphagia symptoms for said consumer when consuming the prepared food product.

The method may further comprise training the set of algorithms to determine a fluid thickness score for the target fluid food product from the received list of amounts of ingredients based on the plurality of fluid thickness scores for the fluid food product recipes in said database in order to gradually improve the accuracy of the method.

The method may further comprise receiving user feedback information from said user interface on at least one of the fluid thickness score and/or a taste score of the target fluid food product produced based on the list of amounts of ingredients including at least one of said alterations to said list; and training the set of algorithms based on the received user feedback in order to gradually improve the accuracy of the method.

In said method, the at least one alteration to the received list of amounts of ingredients may include a change in a ratio of solid and liquid ingredients in said list. Alternatively or additionally, the at least one alteration to the received list of amounts of ingredients may include at least one of a replacement of at least part of an amount of a first liquid ingredient in said received list with a further amount of a further liquid ingredient having a higher thickness score than said first liquid ingredient; and a replacement of at least part of an amount of a first solid ingredient in said received list with a further amount of a further solid ingredient having a higher fat content and/or a higher starch content than said first solid ingredient or an addition of said further amount of the further solid ingredient to said received list of amounts of ingredients.

### BRIEF DESCRIPTION OF THE DRAWINGS

Embodiments of the invention are described in more detail and by way of non-limiting examples with reference to the accompanying drawings, wherein:
Fig. 1 schematically depicts a food processing apparatus according to an embodiment;
Fig. 2 schematically depicts a food processing apparatus according to another embodiment;
Fig. 3 depicts a flowchart of an operating principle of a food processing apparatus according to an embodiment; and
Fig. 4 depicts a flowchart of a method of controlling a food processing apparatus according to an embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.
Embodiments of the present invention provide a food processing apparatus that is configured to receive a recipe of a fluid food product, determine a fluid thickness score of said fluid food product and to adjust said recipe if necessary based on the determined fluid thickness score. The food processing apparatus in typical embodiments is a kitchen appliance for use in a domestic or commercial kitchen, such as a blender, juicer, soup maker or the like although other embodiments of such a food processing apparatus will be immediately apparent to the skilled person.

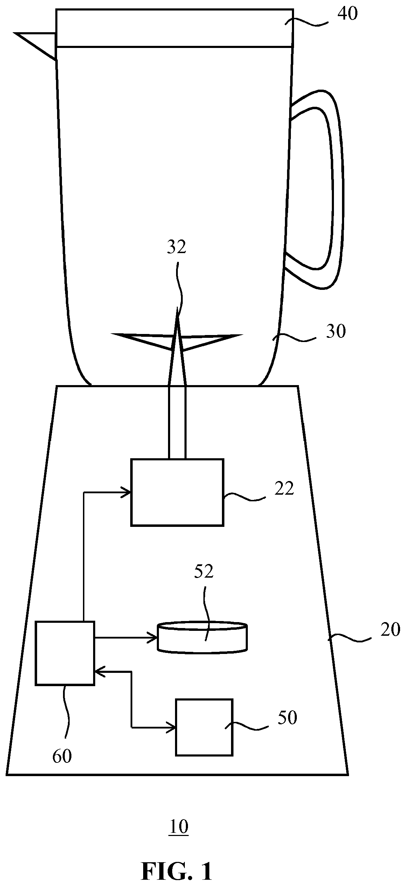

FIG. 1 schematically depicts a cross-sectional view of a food processing apparatus 10 according to an example embodiment of the present invention. In this embodiment, the food processing apparatus 10 may be a blender or the like, but it should be understood that other configurations of the food processing apparatus 10, e.g. when the food processing apparatus 10 is a soup maker or the like, are equally feasible. The food processing apparatus 10 according to the example embodiment comprises a food processing compartment 30, which typically comprises a blade arrangement 32 to grind, macerate or otherwise cut or blend food products. The blade arrangement 32 maybe detachable from the food processing compartment 30, e.g. in order to facilitate cleaning of the blade arrangement 32. The food processing compartment 30 may take any suitable form, such as for example a glass or plastic jug or bowl that may be hermetically sealed by a lid 40. The blade arrangement 32 is driven by a motor 22 under control of a controller 60, which may be housed in a base 20 of the food processing apparatus 10. The motor 22 may be coupled to the blade arrangement in any suitable manner, e.g. through a drive axle or shaft, gear box and so on. Such types of couplings are well-known per se and are therefore not explained in further detail for the sake of brevity only. A heating element (not shown) under control of the controller 60 may be thermally coupled to the food processing compartment 30 such as to heat the contents of the food processing compartment 30, e.g. in the case of the food processing apparatus 10 implementing a soup maker for example. The controller 60 maybe any suitable control arrangement comprising one or more physical entities implementing such a control arrangement.

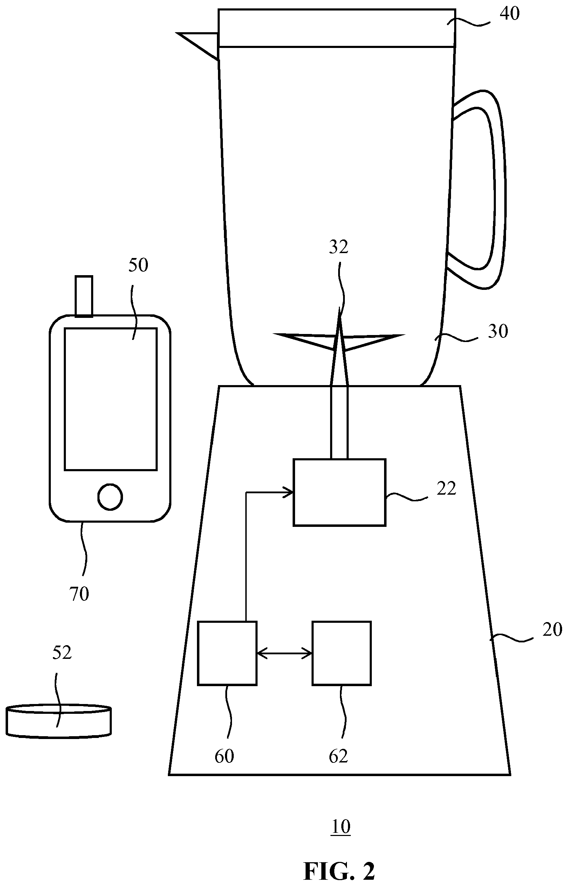

The controller 60 may be responsive to a user interface 50 through which the food processing apparatus 10 may be controlled, e.g. by a user providing the controller 60 with a recipe of a target fluid food product including a list of amounts of ingredients for preparing the target fluid food product. Such a user interface 50 may form part of the food processing apparatus 10, in which case the user interface 50 may be implemented in any suitable manner, e.g. as a touchscreen display, one or more switches, buttons, knobs or dials, and so on, or any combination of such user interface elements. The user interface 50 for example maybe located on the base 20 of the food processing apparatus 10 or in any other suitable location thereon. Alternatively, as schematically depicted in FIG. 2, the user interface 50 maybe implemented on a remote device 70, e.g. by way of a software program such as an app, through which the food processing apparatus 10 maybe remotely controlled. For example, such a remote device 70 may be a computing device, a mobile communication device such as a smart phone, a tablet computer, a remote controller, and so on. In embodiments in which the user interface 50 is implemented on such a remote device, the food processing apparatus 10 typically further comprises a communication interface 62 communicatively coupled to the controller 60, preferably a wireless communication interface through which the remote device may communicate with the food processing apparatus 10. Such a communication link may be a direct (P2P) link such as a Bluetooth link or the like, or may be an indirect link running through a communication management device such as a server, router or the like. As the technology involved with such communication links is well-known per se, this will not be explained in further detail for the sake of brevity only.

The controller 60 is further communicatively coupled to a database 52 storing a plurality of fluid food product recipes, each of said recipes being associated with a fluid thickness score for the fluid food product to be made from said recipe. Such recipes typically include a list of ingredients and the amount of each ingredient in the recipe. The fluid thickness score associated with each database recipe typically is a reliable or accurate fluid thickness score, which for instance may have been determined experimentally, e.g. through measurement and/or may have been verified by one or more persons, e.g. one or more dysphagia sufferers. The fluid thickness score may be expressed in any suitable manner, e.g. in accordance with the IDDSI framework, as a viscosity score, as a rheological parameter, and so on. Any parameter than can be used as an indicator of the thickness of a fluid food product may be used for this purpose. For such a score, a higher value preferably indicates a higher fluid thickness, e.g. as is the case in the IDDSI framework, or alternatively indicates a lower fluid thickness. The database 52 may form part of the food processing apparatus 10, or may be a remote database accessible by the controller 60 through the communication interface 62, as schematically depicted in FIG. 2. Of course, alternative embodiments in which one of the user interface 50 and the database 52 forms part of the food processing apparatus 10 are also contemplated.

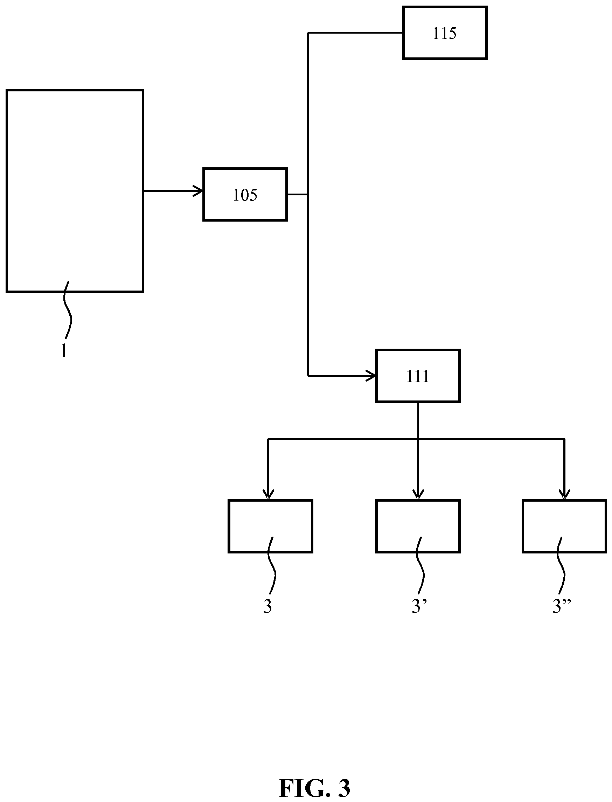

A typical mode of operation of the food processing apparatus 10 is schematically depicted in FIG. 3. In operation, the user of the food processing apparatus 10 provides the controller 60 with a recipe including a list 1 of ingredients and amounts for each ingredient in said list through the user interface 50. The recipe may be selected by the user from a list of recipes available through the user interface 50 or alternatively the user may manually input the list 1 of ingredients through the user interface, e.g. from scratch or by amending a recipe from the list of ingredients. The way the user generates the list 1 of ingredients is not particularly limited and any suitable manner of providing such a user input may be used. The user may further provide an indication of the desired (target) fluid thickness score of the fluid food product to be produced. To this end, the user may provide a particular target fluid thickness score, e.g. the fluid thickness score for said user for which the dysphagia symptoms of the user are minimized, or instead the user may provide a user identifier, e.g. a user name, a biometric identifier such as a scanned fingerprint or the like, in which case the controller 60 may have access to a further database (not shown) storing a plurality of such user identifiers, which each user identifier being associated with a target fluid thickness score such that the controller can determine the target thickness score of a particular user by matching his or her user identifier as provided through the user interface 50 with a user identifier in the further database and retrieving the target fluid thickness score associated with the matched user identifier in the further database. Such a further database may form part of the food processing apparatus 10 or maybe a remote entity accessible through the communication interface 62 as previously explained.

In operation 105, the controller 60 determines a fluid thickness score for the target fluid food product as defined by the list 1 of ingredients based on a comparison of the received list of amounts of ingredients with at least some of the fluid food product recipes in the database 52. For example, if the recipe as specified by the user matches one of the recipes in the database 52, the fluid thickness score associated with the database recipe can be directly used for a comparison against the target fluid thickness score for the user, as such a database fluid thickness score is a known good score as previously explained. Alternatively, if the recipe as specified by the user does not match any of the recipes in the database 52, the controller 60 may estimate a fluid thickness score for the food product to be prepared from the user specified recipe based on a similarity to one or more recipes in the database 52 and a knowledge of the thickening characteristics of particular ingredients. For example, where a user specifies a recipe having a liquid such as water as one of the ingredients, a difference between the amount of water in the user specified recipe and the amount of water in a similar recipe in the database 52 may be used by the controller 60 to adjust the fluid thickness score of the database recipe based on the difference between the respective amounts of water in order to estimate or extrapolate the fluid thickness score of the user specified recipe. As another example, a different amount of fattening or binding ingredient between a user specified recipe and a similar database recipe may be used by the controller 60 to estimate or extrapolate the fluid thickness score of the user specified recipe from the fluid thickness score of the database recipe. It will be understood that these are non-limiting examples of how the controller 60 may estimate a fluid thickness score of a user specified recipe, and many more strategies will be immediately apparent to the skilled person. For example, the controller 60 may evaluate a plurality of recipes in the database 52 that each have a similarity with the user specified recipe in order to estimate a fluid thickness score of the user specified recipe based on trends or overlaps between said plurality of recipes and/or differences between each database recipe and the user specified recipe. In order to determine the fluid thickness score of a user specified recipe, the controller 60 typically implements a set of algorithms, which may be trained in at least some embodiments to improve the fluid thickness score determination of a user specified recipe as will be explained in more detail below.

Upon determination of the fluid thickness score of the user specified recipe, the controller 60 compares the determined fluid thickness score for the target fluid food product against a critical fluid thickness score for the user specifying the recipe, that is, the fluid thickness score for which the dysphagia symptoms of the user are minimal. If this comparison is indicative of the fluid thickness of the target fluid food product as specified by the user recipe is below a critical thickness for the target fluid food product, the controller 60 in operation 111 suggests one or more amendments 3, 3', 3" to the user specified recipe in order to increase the expected fluid thickness of the fluid food product to be prepared. The one or more suggested amendments 3, 3', 3" to the user specified recipe may be presented to the user on the user interface 50, for example. Such amendments for example may involve one or more of a change in a ratio of solid and liquid ingredients in said list, a replacement of at least part of an amount of a first liquid ingredient in said received list with a further amount of a further liquid ingredient having a higher thickness score than said first liquid ingredient, such as replacing water with yoghurt for example; a replacement of at least part of an amount of a first solid ingredient in said received list with a further amount of a further solid ingredient having a higher fat content and/or a higher starch content than said first solid ingredient, and an addition of said further amount of the further solid ingredient to said received list of amounts of ingredients in order to increase the expected fluid thickness score of the food product to be prepared. Starch-rich ingredients include banana, pumpkin, potato and lentils for example, whereas fat-rich ingredients include avocado for example.

Preferably, the suggested amendments to the user specified recipe are chosen such as to minimize the change to the taste of the food product to be prepared. To this end, the controller 60 may include or have access to a list of ingredients and suitable replacements thereof, such that the controller 60 may recommend one or more amendments to the user specified recipe based on this list of ingredients. For example, the list may indicate a number of suitable replacements of liquid and solid ingredients based on an increased fat or starch content of the replacement ingredients compared to the originally specified ingredient in the user specified recipe. In this manner, the user may adjust his or her recipe based on at least some of the one or more amendments 3, 3', 3" to the recipe as suggested by the controller 60 prior to loading the ingredients into the food processing compartment 30 of the food processing apparatus 10 and subsequently preparing the fluid food product. If on the other hand the controller 60 determines that the determined fluid thickness score of the target fluid food product to be prepared in accordance with the user specified recipe at least matches the critical thickness for the target fluid food product, the controller 60 may directly invoke operation 115 in which processing of the target fluid food product may be initiated after the user has loaded the ingredients in accordance with the user specified recipe into the food processing apparatus 10.

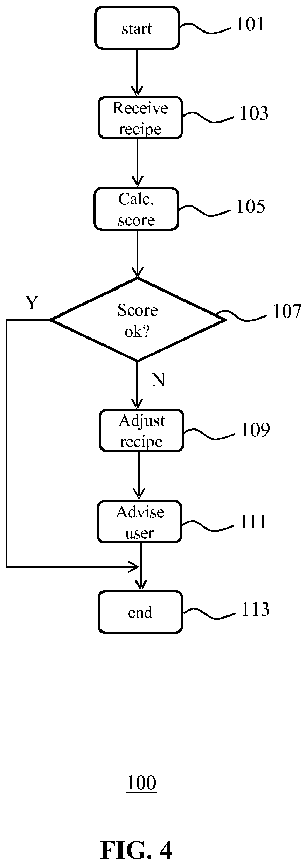

FIG. 4 is a flowchart of a method 100 of controlling the food processing apparatus 10. After the method 100 starts in operation 101, e.g. by switching on the food processing apparatus 10, the method 100 proceeds to operation 103 in which the controller 60 receives the user specified recipe including the list 1 of ingredients and their amounts through the user interface 50. In operation 105, the controller 60 determines the fluid thickness score of the food product to be prepared in accordance with the user specified recipe as explained in more detail above, and checks in operation 107 if the determined fluid thickness score of this food product at least matches the critical fluid thickness score associated with the user providing the recipe, which critical fluid thickness score as previously explained for instance may be provided by the user through the user interface 50 or may be associated with a user identifier in a database of user identifiers such that the controller 60 may retrieve the critical fluid thickness score from this database upon receiving a user identifier through the user interface 50.

If it is determined in operation 107 that the determined fluid thickness score of the food product to be prepared in accordance with the user specified recipe at least matches the critical fluid thickness score associated with the user providing the recipe, the method 100 may terminate in operation 113 as no adjustment of this recipe is required. However, if it is determined in operation 107 that the determined fluid thickness score of the food product to be prepared in accordance with the user specified recipe is below the critical fluid thickness score associated with the user providing the recipe, the method 100 proceeds to operation 109 in which the controller 60 generates at least one amendment to the user specified recipe as previously explained in order to increase the fluid thickness score of the fluid food product to be prepared in accordance with the user specified recipe and communicates the at least one determined alteration to the received list 1 of amounts of ingredients to the user interface 50 in operation 111 prior to terminating in operation 113. In this manner, a food processing apparatus 10 is provided that assists a user in preparing a food product having an appropriate fluid thickness score for that user, that is, a fluid thickness score for which the user's dysphagia symptoms are minimized.

As previously mentioned, in order to determine the fluid thickness score of a fluid food product to be prepared in accordance with a user specified recipe, the controller 60 typically implements a set of algorithms including at least one algorithm, which set of algorithms preferably may be trained in order to improve the quality and/or accuracy of the fluid thickness score determination process and/or the recipe adjustment process. For example, the set of algorithms may comprise an algorithm for determining the fluid thickness score of a fluid food product to be prepared in accordance with a user specified recipe that uses the database 52 of recipes for food products having known reliable fluid thickness scores, e.g. because these scores have been established in controlled experiments such as laboratory tests, as a training set in order to establish knowledge of the impact of the (amounts of) various ingredients in such recipes on the fluid thickness score of a food product comprising a particular amount of such an ingredient. For example, the algorithm may train itself to establish how a liquid/solid ratio should be changed, how much of a specified amount of a particular liquid in a recipe should be replaced by a further amount of a thicker liquid, how much starch-rich or fat-rich food should be added to the recipe, and so on, in order to cause a desired change in the fluid thickness score of a particular food product.

Such an algorithm may be further developed or refined by the addition of new recipes with associated fluid thickness scores to the database 52, by the provision of experimental data for augmented recipes, and/or by the provision of user feedback, e.g. through the user interface 50, on the accuracy of the fluid thickness score of a food product as determined by this algorithm. Such user feedback for instance may be an indication of the prepared food product having insufficient fluid thickness or having a higher than expected fluid thickness, which information may be used in the algorithm training to further refine the fluid thickness score determination process. Similarly, the algorithm may be trained based on user feedback pertaining to the perceived change in taste of the food product prepared in accordance with a recipe augmented as per a suggestion generated by the controller 60, such that the algorithm can learn which alterations to a recipe are particularly suitable for increasing the fluid thickness score of a food product without significantly affecting its taste. As such algorithm training, e.g. machine learning, is now well-known per se, this will not be further explained for the sake of brevity only.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A food processing apparatus (10) for preparing a target fluid food product comprising:
a controller (60) for controlling the food processing apparatus, said controller in operation being responsive to a user interface (50) for said food processing apparatus and having access to database (52) storing a plurality of fluid food product recipes, each of said recipes being associated with a fluid thickness score for the fluid food product to be made from said recipe, the controller implementing a set of algorithms configured to:
receive (103) a list (1) of amounts of ingredients for preparing the target fluid food product from the user interface;
determine (105) a fluid thickness score for the target fluid food product based on a comparison of the received list of amounts of ingredients with at least some of the fluid food product recipes in said database;
compare (107) the determined fluid thickness score for the target fluid food product against a critical fluid thickness score, and, if said comparison is indicative of the fluid thickness of the target fluid food product being below a critical thickness for said target fluid food product:
determine (109) at least one alteration (3, 3', 3") to the received list of amounts of ingredients for increasing the fluid thickness score of the target fluid food product to at least the critical fluid thickness score; and
communicate (111) the at least one determined alteration to the received list of amounts of ingredients to the user interface.

2. The food processing apparatus (10) of claim 1, wherein the controller (60) is further configured to train the set of algorithms to determine a fluid thickness score for the target fluid food product from the received list of amounts of ingredients based on the plurality of fluid thickness scores for the fluid food product recipes in said database (52).

3. The food processing apparatus (10) of claim 1 or 2, wherein the controller (60) is further configured to:
receive user feedback information from said user interface (50) on at least one of the fluid thickness score and a taste score of the target fluid food product produced based on the list of amounts of ingredients including at least one of said alterations to said list; and
train the set of algorithms based on the received user feedback.

4. The food processing apparatus (10) of any of claims 1-3, wherein the at least one alteration (3, 3', 3") to the received list (1) of amounts of ingredients includes a change in a ratio of solid and liquid ingredients in said list.

5. The food processing apparatus (10) of any of claims 1-4, wherein the at least one alteration (3, 3', 3") to the received list (1) of amounts of ingredients includes at least one of:
a replacement of at least part of an amount of a first liquid ingredient in said received list with a further amount of a further liquid ingredient having a higher thickness score than said first liquid ingredient; and
a replacement of at least part of an amount of a first solid ingredient in said received list with a further amount of a further solid ingredient having a higher fat content and/or a higher starch content than said first solid ingredient or an addition of said further amount of the further solid ingredient to said received list of amounts of ingredients.

6. The food processing apparatus (10) of any of claims 1-5, wherein the controller (60) is further adapted to directly or indirectly obtain said critical fluid thickness score from the user interface (50).

7. The food processing apparatus (10) of claim 6, further comprising a further database of user identifiers each associated with a critical fluid thickness score for said user, wherein the controller (60) is adapted to retrieve the critical fluid thickness from said further database based on a user identifier received from the user interface (50).

8. The food processing apparatus (10) of any of claims 1-7, further comprising said user interface (50).

9. The food processing apparatus (10) of any of claims 1-8, further comprising said database (52).

10. The food processing apparatus (10) of any of claims 1-9, further comprising a communication interface (62) communicatively coupled to the controller (60) for communicating with at least one of the user interface (50) and the database (52).

11. A method (100) of controlling a food processing apparatus (10) for preparing a target fluid food product, the method comprising:
receiving (103) a list (1) of amounts of ingredients for preparing the target fluid food product from a user interface (50) for said food processing apparatus;
determining (105) a fluid thickness score for the target fluid food product based on a comparison of the received list of amounts of ingredients with at least some fluid food product recipes in a database (52) storing a plurality of said fluid food product recipes, each of said recipes being associated with a fluid thickness score for the fluid food product to be made from said recipe;
comparing (107) the determined fluid thickness score for the target fluid food product against a critical fluid thickness score, and, if said comparison is indicative of the fluid thickness of the target fluid food product being below a critical thickness for said target fluid food product:
determining (109) at least one alteration (3, 3', 3") to the received list of amounts of ingredients for increasing the fluid thickness score of the target fluid food product to at least the critical fluid thickness score; and
communicating (111) the at least one alteration to the received list of amounts of ingredients to the user interface.

12. The method (100) of claim 11, further comprising training the set of algorithms to determine a fluid thickness score for the target fluid food product from the received list (1) of amounts of ingredients based on the plurality of fluid thickness scores for the fluid food product recipes in said database (52).

13. The method (100) of claim 11 or 12, further comprising:
receiving user feedback information from said user interface (50) on at least one of the fluid thickness score and/or a taste score of the target fluid food product produced based on the list (1) of amounts of ingredients including at least one of said alterations to said list; and training the set of algorithms based on the received user feedback.

14. The method (100) of any of claims 11-13, wherein the at least one alteration (3, 3', 3") to the received list (1) of amounts of ingredients includes a change in a ratio of solid and liquid ingredients in said list.

15. The method (100) of any of claims 11-14, wherein the at least one alteration (3, 3', 3") to the received list (1) of amounts of ingredients includes at least one of:
a replacement of at least part of an amount of a first liquid ingredient in said received list with a further amount of a further liquid ingredient having a higher thickness score than said first liquid ingredient; and
a replacement of at least part of an amount of a first solid ingredient in said received list with a further amount of a further solid ingredient having a higher fat content and/or a higher starch content than said first solid ingredient or an addition of said further amount of the further solid ingredient to said received list of amounts of ingredients.
